# EUROPEAN PATENT APPLICATION

(11) **EP 3 327 675 A1**
(43) Date of publication of application: **30.05.2018**
(21) Application number: 16827938.8
(22) Date of filing: 21.06.2016
(51) Int. Cl.: G06T 17/00, B29C 67/00, B33Y 30/00, B33Y 50/00, G01B 11/24, G01B 11/245

(54) **MEDICAL AID MANUFACTURING SYSTEM AND METHOD**

(30) Priority: 23.07.2015 KR 20150104565
(71) Applicant: Solideng Co., Ltd., Daejeon 34068 (KR); Samsung Life Public Welfare Foundation, Seoul 04348 (KR)
(72) Inventor: YU, Hong Jong, Seongnam-si Gyeonggi-do 13527 (KR); JOO, Il Won, Ansan-si Gyeonggi-do 15621 (KR); KIM, Sang Jun, Seoul 135-710 (KR)
(74) Representative: Nobbe, Matthias
(86) International application number: PCT/KR2016/006575
(87) International publication number: WO 2017/014434

(57) **Abstract**

The present invention provides an orthosis design manufacturing system, comprising: an orthosis design generating system configured: to generate an information of a reference point using mark points displayed on a 3D image of an outer form for a body part of an examinee on which an orthosis is worn; to generate a reference model including an outer form information of the 3D image and the information of the reference point; and based on the information of the reference point, to map a selected orthosis design template to the reference model and generate an orthosis design, wherein the information of the reference point includes an information of a joint reference point for a joint axis of the body part.

## Description

### [Technical Field]

The present invention relates to system and method of manufacturing orthosis

### [Background Art]

When manufacturing an orthosis used as a device to assist or correct a human body function, following processes are conducted in the prior art.

In other words, the prior art orthosis is manufactured by processes: scanning a body part on which an orthosis is worn using a 3D scanner and generating 3D image data; then correcting the 3D image by a worker and generating a body part design; then manufacturing a processed product for the body part design using an NC (numerical control work) manufacturing apparatus; and then attaching an orthosis producing material on an outer surface of the processed product and making a model.

However, the prior art orthosis manufacturing have some problems.

In the prior art, since many processes, including manufacturing the processed product for the body part and making the model for the processed product, are accompanied, there is a problem that a manufacturing time becomes very long.

Further, the 3D image data are merely data for surfaces of the corresponding body part and do not include an information of a skeleton structure including joints that have a close relation to a use of the orthosis. Accordingly, there is a limit in manufacturing an orthosis suitable to a patient.

Further, since generating the body part design is totally dependent on the worker's experience, there is a problem that variation happens according to workers and it is difficult to secure a reliability of a product. Further, since a process of making the model manually to manufacture the orthosis is conducted, there similarly happens a problem that it is difficult to secure a reliability of a product.

As such, since a reliability of a product is not secured, if an orthosis is not suitable after it is worn by a patient, a problem is caused that cumbersome manufacturing processes are conducted again.

As above, according to the prior art orthosis manufacturing method, there happen problems such as a product efficiency being reduced and a product reliability not being secured.

### [Detailed Description of Invention]

### [Technical Problem]

An object of the present invention is to provide a solution that can improve an orthosis manufacturing efficiency and secure reliability.

### [Technical Solution]

To achieve the above object, the present invention provides an orthosis design manufacturing system, comprising: an orthosis design generating system configured: to generate an information of a reference point using mark points displayed on a 3D image of an outer form for a body part of an examinee on which an orthosis is worn; to generate a reference model including an outer form information of the 3D image and the information of the reference point; and based on the information of the reference point, to map a selected orthosis design template to the reference model and generate an orthosis design, wherein the information of the reference point includes an information of a joint reference point for a joint axis of the body part.

Here, the mark points may include a first mark point that is marked directly on the body part and is displayed on the 3D image, and a second mark point that is marked on the 3D image using the orthosis design generating system and is displayed on the 3D image.

The orthosis design template may include an information of a standard reference point corresponding to the information of the reference point, and the standard reference point may be matched to the reference point of the reference model, and based on this matching, the orthosis design template may be mapped to the reference model.

When matching a standard joint reference point to a joint reference point of the reference model, a joint axis of the standard joint reference point may be matched to a joint axis of the joint reference point.

The reference point may include an origin point of a coordinate system for the body part.

The orthosis design generating system may be configured to map the orthosis design template to the reference model and generate the orthosis design, based on an information of a bone line connecting the reference points neighboring to each other and the information of the reference point.

The orthosis design generating system may be configured to correct a posture of the reference model.

The orthosis design generating system may be configured to adjust a thickness for at least a region of the orthosis design.

The body part may be one of four limbs or a neck.

The orthosis design manufacturing system may further comprise: a 3D scanner that scans the body part and generates the 3D image; and a 3D printer that receives an information of the orthosis design and manufactures the orthosis.

In another aspect, the present invention provides an orthosis design manufacturing method, comprising: in an orthosis design generating system, generating an information of a reference point using mark points displayed on a 3D image of an outer form for a body part of an examinee on which an orthosis is worn; generating a reference model including an outer form information of the 3D image and the information of the reference point; and based on the information of the reference point, mapping a selected orthosis design template to the reference model and generating an orthosis design, wherein the information of the reference point includes an information of a joint reference point for a joint axis of the body part.

Here, the mark points may include a first mark point that is marked directly on the body part and is displayed on the 3D image, and a second mark point that is marked on the 3D image using the orthosis design generating system and is displayed on the 3D image.

The orthosis design template may include an information of a standard reference point corresponding to the information of the reference point, and the standard reference point may be matched to the reference point of the reference model, and based on this matching, the orthosis design template may be mapped to the reference model.

When matching a standard joint reference point to a joint reference point of the reference model, a joint axis of the standard joint reference point may be matched to a joint axis of the joint reference point.

The reference point may include an origin point of a coordinate system for the body part.

In the orthosis design generating system, the orthosis design template may be mapped to the reference model and the orthosis design may be generated, based on an information of a bone line connecting the reference points neighboring to each other and the information of the reference point.

In the orthosis design generating system, a posture of the reference model may be corrected.

In the orthosis design generating system, a thickness for at least a region of the orthosis design may be adjusted.

The body part may be one of four limbs or a neck.

The orthosis design manufacturing method may further comprise: scanning the body part and generating the 3D image using a 3D scanner; and receiving an information of the orthosis design and manufacturing the orthosis in a 3D printer.

### [Advantageous Effects]

According to the present invention, the reference model is generated by setting the reference points that become a reference of a joint structure or skeleton structure of a body part on which the orthosis is worn, the orthosis design is generated by mapping the orthosis design template to the reference model based on the reference points, and the orthosis can be manufactured just with the generated orthosis design.

Accordingly, the orthosis optimized to the orthosis wearer can be manufactured, and thus a reliability of manufacturing the orthosis can be maximized.

Further, since the orthosis design can be generated in a very short time through the orthosis design generating application and then the orthosis can be manufactured immediately using the orthosis manufacturing apparatus, manufacturing processes of the orthosis can be reduced and simplified considerably and thus an orthosis manufacturing efficiency can be maximized.

### [Brief Description of Drawings]

FIG. 1 is a schematic view illustrating a system of manufacturing an orthosis according to an embodiment of the present invention.
FIG. 2 is a schematic flow chart illustrating a method of manufacturing an orthosis according to an embodiment of the present invention.
FIG. 3 is a schematic view illustrating mark points displayed on a nether extremity image according to an embodiment of the present invention.
FIGs. 4 to 7 are schematic views illustrating setting several reference points of a nether extremity using mark points according to an embodiment of the present invention.
FIG. 8 is a schematic view illustrating setting reference points and a bone line of a nether extremity according to an embodiment of the present invention.
FIG. 9 is a schematic view illustrating an orthosis design that is mapped to a reference model generated according to an embodiment of the present invention and is formed.
FIG. 10 is a schematic view illustrating adjusting a thickness of an orthosis design generated according to an embodiment of the present invention.

### [Mode for Embodying Invention]

Embodiments of the present invention are explained with reference to Figures.

Orthosis manufactured according to the present invention may be an orthosis used for a human and animal as well, and further, may be any one of all types of orthoses which are worn on all body organs. For the convenience of explanations, the below embodiments explain an orthosis worn on a nether extremity (a leg) of a human by way of example.

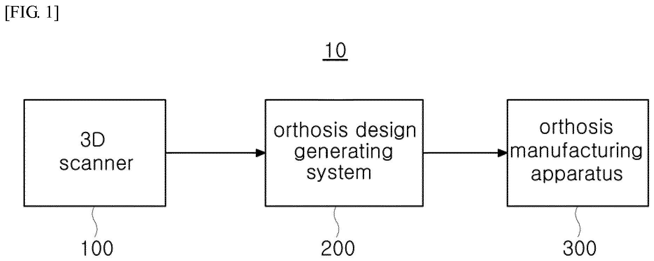

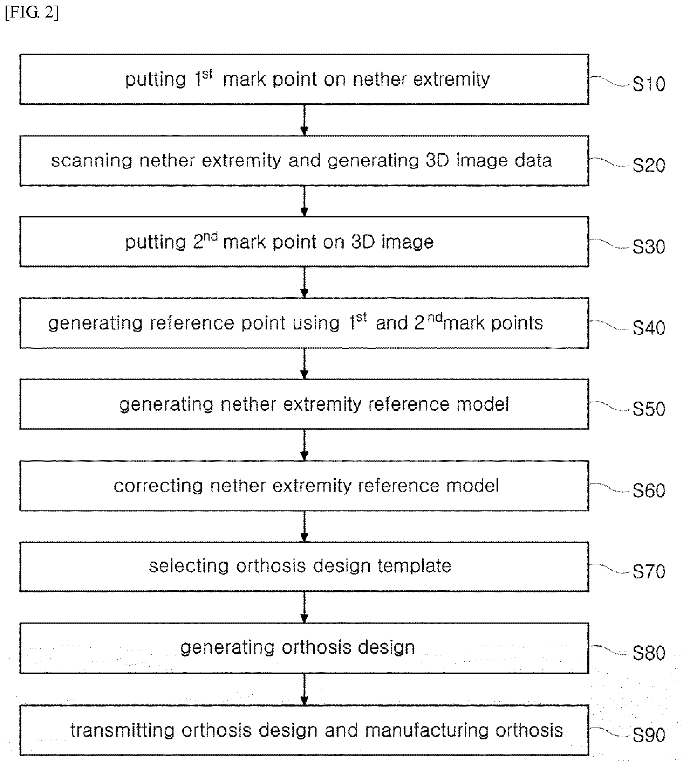

FIG. 1 is a schematic view illustrating a system of manufacturing an orthosis according to an embodiment of the present invention, and FIG. 2 is a schematic flow chart illustrating a method of manufacturing an orthosis according to an embodiment of the present invention.

Referring to FIG. 1, a system 10 of manufacturing an orthosis according to an embodiment of the present invention may include a 3D scanner 100, an orthosis design generating system 200, and an orthosis manufacturing apparatus 300.

The orthosis manufacturing system including such the configuration and the orthosis manufacturing method using the same are explained together below.

The 3D scanner 100 is a component that conducts scanning a body part, for example, a nether extremity on which the orthosis is worn, and generates and outputs 3D image data for an outer form of the nether extremity (S20).

At this time, according to the embodiment of the present invention, before scanning the nether extremity using the 3D scanner 100, while a medical staff, such as a doctor, really touching the nether extremity of a wearer who will wear the orthosis, the medical staff may conduct marking on a skin of a joint part i.e., a skin of a joint axis of the nether extremity using a marking pen or the like, and thus a mark point i.e., a first mark point may be put (S10).

In this regard, there are several joints serving rotation functions at the nether extremity, and a skin of at least a part out of the several joints may be marked. In this embodiment, skins of parts associated with a knee joint, a talocrural joint, as a joint associated with rotation of an ankle, which is associated with an up and down rotation, and a subtalar joint, as a joint associated with rotation of an ankle, which is associated with right and left rotation, are marked, and thus the first mark points for the joint axes are put.

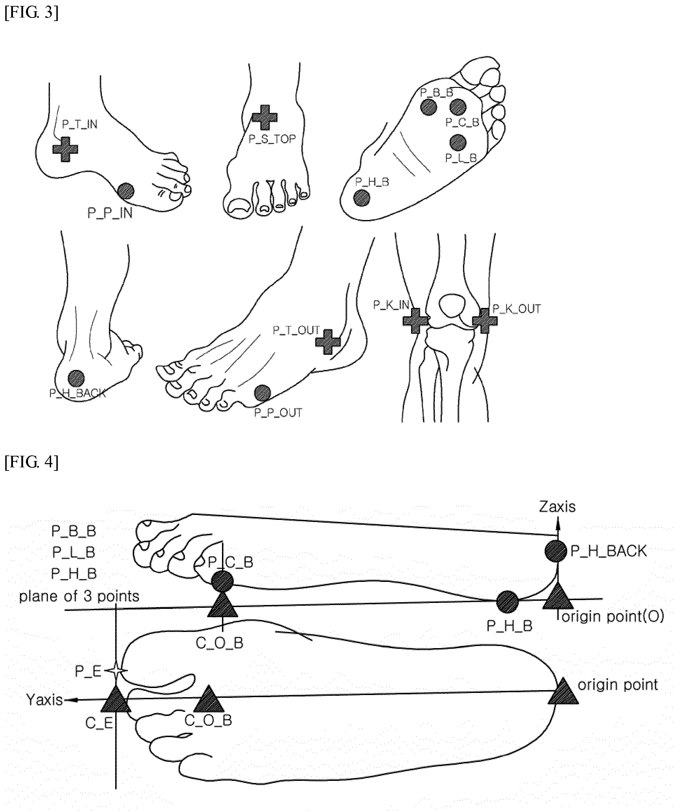

Putting the first mark points refers to FIG. 3, and for the convenience of explanations, the first mark point is indicated with a cross (+) shape. Referring to this, first mark points P_K_IN and P_K_OUT for an inner side end and an outer side end of the knee joint, first mark points P_T_IN and P_T_OUT for an inner side end and an outer side end of the talocrural joint, and a first mark points P_S_TOP for an end of a top of a foot (an end of a dorsum of foot) of the subtalar joint are shown.

In the state that the first mark points are put, the 3D scanning for the nether extremity is conduct, and thus the 3D image data include an information of the first mask points.

The orthosis design generating system 200 is a component that is input with the 3D image data which are output from the 3D scanner 100, and generates an orthosis design for the nether extremity using the 3D image data.

A computer may be used as the orthosis design generating system 200, and this system 200 may include a mainframe which executes an orthosis design generating application and performs an operation process, a monitor of an image output device to display images, and an input device, such as a keyboard, mouse or the like, processing a worker's input action.

The orthosis design generating system 200 displays a scanning image, which is a nether extremity source model for an orthosis wearing, on the monitor. In the scanning image displayed on the monitor, the first mark points are displayed together.

To set up reference points for generating the orthosis design suitable to the nether extremity, the worker may mark at specific positions on the scanning image (i.e., at specific positions on the skin) and generate second mark points (S30). Using an information of the second mark points and the information of the first mark points, which exist on the scanning image, together, the reference points to generate a suitable orthosis design are generated (S40). Meanwhile, by the reference points, a reference bone line may be generated.

The reference points may include, for example, an origin point of a coordinate system to express each part of the nether extremity with the coordinate system and a joint reference point to specify a joint axis, and further, may include a tiptoe reference point that indicates an end of toes as an end of the nether extremity that is an end of such the body part. Further, by connecting neighboring reference points, the reference bone line may be defined.

This is explained in more detail with reference to drawings.

First, referring back to FIG. 3 regarding putting the second mark points, the second mark points are indicated with a dot shape. A second mark point P_H_BACK for a back end of a heel, a second mark point P_H_B for a bottom end of a heel, a second mark point P_B_B for a bottom end of a metatarsophalangeal joint of a big toe, a second mark point P_L_B for a bottom end of a metatarsophalangeal joint of a little toe, a second mark point P_C_B for a bottom end of a metatarsophalangeal joint of a second toe, a second mark point P_P_IN for an inner side end of a metatarsophalangeal joint of a big toe, and a second mark point P_P_OUT for an outer side end of a metatarsophalangeal joint of a little toe are shown.

Then, referring to FIG. 4 regarding generating the origin point of the coordinate system, a line that is normal to a plane (i.e., a plane of a sole) passing through the point P_H_B for the bottom end of the heel, the point P_B_B for the bottom end of the metatarsophalangeal joint of the big toe and the point P_L_B for the bottom end of the metatarsophalangeal joint of the little toe and that passes through the point P_H_BACK for the back end of the heel may be defined as a Z axis of the origin point O of the coordinate system, and a point of the plane of the sole meeting the Z axis line may be defined as the origin point O. Further, a line that connects the origin point O of the coordinate system with a point C_O_B at which a line passing through the point P_C_B for the bottom end of the metatarsophalangeal joint of the second toe in a vertical direction (i.e., the Z axis direction) meets the plane of the sole may be defined as a Y axis, and thus a X axis perpendicular to the Y axis and the Z axis may be defined.

Meanwhile, a point, at which a plane that passes through an endmost point P_E of the toes and that is perpendicular to the Y axis meets the Y axis, is defined as a foot end reference point C_E. The endmost point P_E of the toes may be displayed on the image by the worker or be detected automatically by an image process.

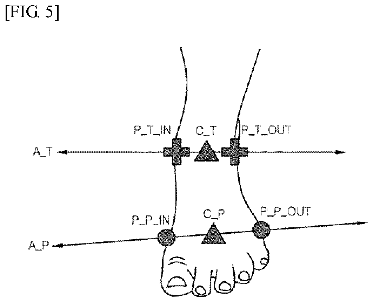

Then, referring to FIG. 5 regarding generating a reference point for a talocrural joint axis, a center point C_T of a talocrural joint axis A_T that is a line connecting the inner side end point P_T_IN and the outer side end point P_T_OUT of the talocrural joint is defined as a reference point C_T for this joint axis.

Then, referring to FIG. 5 regarding generating a reference point for a toe joint axis, a center point C_P of a toe joint axis A_P that is a line connecting the inner side end point P_P_IN of the metatarsophalangeal joint of the big toe and the outer side end point P_P_OUT of the metatarsophalangeal joint of the little toe is defined as a reference point C_P for this joint axis.

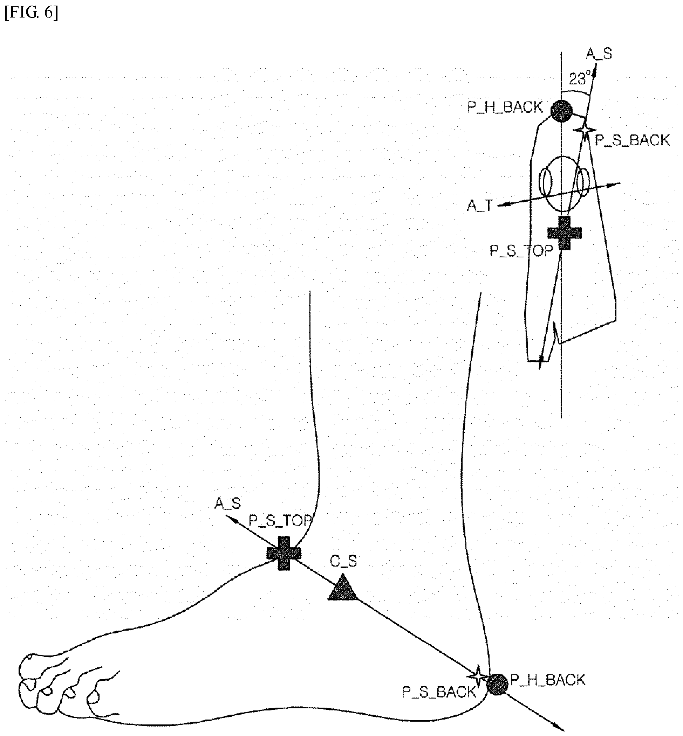

Then, referring to FIG. 6 regarding generating a reference point for a subtalar joint axis, a line connecting the foot top end point P_S_TOP of the subtalar joint and the back end point P_H_BACK of the heel is rotated in a clockwise direction of FIG. 6 (i.e., in an outer side direction from the heel) by a specific angle, for example, 23 degrees on the XY plane of the coordinate system with respect to the foot top end point P_S_TOP, and this line is defined as a subtalar joint axis A_S. At this time, a point P_S_BACK at which the subtalar joint axis A_S meets a back surface of the heel is also defined, and a point, a distance from which to the foot top end point P_S_TOP of the subtalar joint and a distance from which to the point P_S_BACK at which the subtalar joint axis A_S meets a back surface of the heel have a ratio of 3:7, is defined as a reference point C_S of the subtalar joint axis A_S.

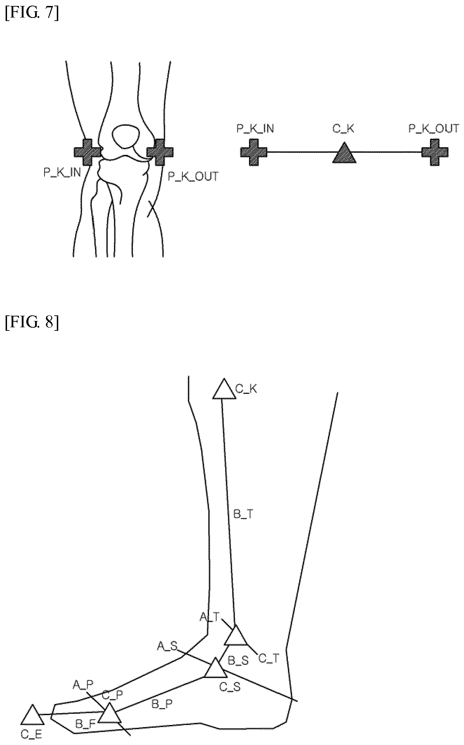

Then, referring to FIG. 7 regarding generating a reference point for a knee joint axis, a center point C_T of a knee joint axis A_T that is a line connecting the inner side end point P_K_IN and the outer side end point P_K_OUT of the knee joint is defined as a reference point C_T for this joint axis.

The above reference points are major reference points associated with a joint structure and a bone structure i.e., a skeleton structure, and through the reference points, the nether skeleton structure can be figured out and specified.

In this regard, for example, as shown in FIG. 8, a connection line that connects the reference point C_K of the knee joint axis and the reference point C_T of the talocrural joint axis may be generated and be defined as a shinbone (tibia) line B_T, a connection line that connects the reference point C_T of the talocrural joint axis A_T and the reference point C_S of the subtalar joint axis A_S may be generated and be defined as a tarsal bone line B_S, a connection line that connects the reference point C_S of the subtalar joint axis A_S and the reference point C_P of the toe joint axis A_P may be generated and be defined as a metatarsal bone line B_P, and a connection line that connects the reference point C_P of the toe joint axis A_P and the foot end reference point C_E may be generated and be defined as a foot phalangeal bone line B_F.

Meanwhile, regarding the first and second mark points to generate the reference points, a number and positions of the first mark points and the second mark points described above are explained by way of example and may be appropriately adjusted or alter as needed. For example, at least a part of the second mark points described above may be replaced with mark points, and similarly, at least a part of the first mark points described above may be replaced with second mark points.

The orthosis design generating system 200 generates a reference point information defined based on the first and second mark points and combines the reference point information with an outer form information of the 3D image, and can generate a nether extremity reference model that becomes a reference of an orthosis design generation.

Meanwhile, informations constituting the nether extremity reference model may further include a reference bone line information along with the reference point information.

Here, the reference point information may include a position of a reference point, and in case that a reference point is a joint reference point, the reference point information may also include an information of a direction of the joint axis (i.e., a vector information).

Meanwhile, after generating the nether extremity reference model, the orthosis design generating system 200 may correct this reference model (S60). In this regard, when scanning, according to a physical deformity or environment, a posture of an examinee is more likely to be inconsistent other than desired, and further, an alteration of a posture may be needed according to a degree of a correction of a body part or the like. In these cases, correction for a nether extremity reference model into a preferred posture is performed.

After generating the nether extremity reference model or performing correction for it, the orthosis design generating system 200 selects a desired orthosis design sample i.e., template (S70). In this regard, since various forms of orthoses may be used according to purpose of use, body form or the like, various forms of orthosis design templates may be prepared and stored beforehand in the orthosis design generating system 200, and among these templates, a design template of a form judged to be suitable to a wearer may be selected. Meanwhile, in selecting the orthosis design template, it is preferred that a prescription of a medical staff is considered.

When the orthosis design template is selected, the orthosis design generating system 200 may modify the selected orthosis design template into a form suitable to the nether extremity reference model and generate an orthosis design (S80).

In this regard, as the orthosis design template is generated based on a standard model for a nether extremity, the orthosis design template may include, for example, a reference point information of the standard model i.e., a standard reference point information, and a standard form information based on standard reference points. Of course, the orthosis design template may include a standard skeleton structure information including the standard reference point information.

Accordingly, matching the standard reference points to reference points of a corresponding reference model is conducted, and based on this, mapping a standard form to an outer form of a reference model, thus an orthosis design template is modified into a form suitable to the reference model and an orthosis design for the reference model can be generated. Here, in matching the reference points, it is preferred that regarding a joint reference point, a joint axis is matched together.

Of course, in case that a standard bone line information is included, a bone line along with a reference point is matched.

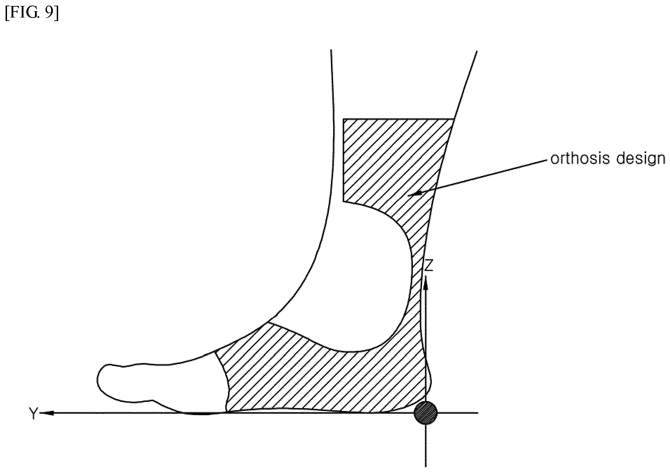

The orthosis design generation refers to FIG. 9, and by mapping an orthosis design template for a nether extremity reference model, an orthosis design can be generated. Particularly, since the orthosis design can be generated through a data process using an orthosis design application, all shapes including a flexible curve or the like that is difficult to realize through a manual task can be freely realized, and thus an orthosis design optimized ergonomically can be easily generated.

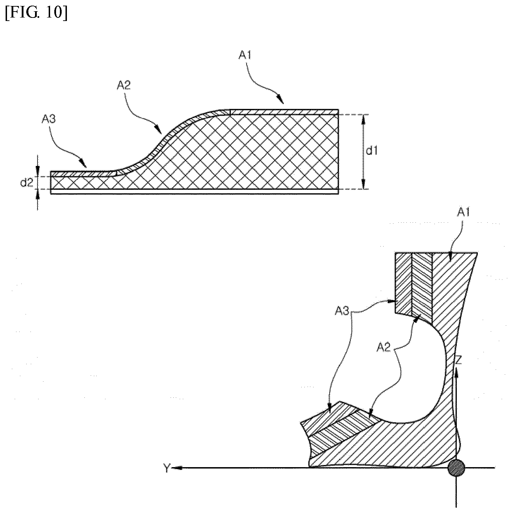

Meanwhile, according to an embodiment of the present invention, the orthosis design generating system 200 may be configured to adjust a thickness for at least a region. In this regard, by way of example with reference to FIG. 10, an orthosis design has a default thickness d1 that is set up at base. When a worker selects a specific region A3 for reduction of thickness, this region A3 may be set to have a thickness d2 less than the default thickness d1, and the thickness d2 of this region A3 may be set by the worker. Meanwhile, if the selected region A3 and a basic region A1 of the default thickness d1 are located to directly adjoin each other, a thickness changes rapidly between these regions and thus a defect of an orthosis or inconvenience of a wearer may be caused. Thus, a buffer region A2 may be set at a predetermined width such that a thickness changes continuously and smoothly between the selected region A3 and the basic region A1, and thus the thickness may change in a curved form at the buffer region A2.

When the orthosis design is generated as above, the orthosis design generating system 200 transmits orthosis design 3D data to the orthosis manufacturing apparatus 300, and the orthosis manufacturing apparatus 300 manufactures an orthosis according to the transmitted orthosis design data (S90). At this time, it is preferred, but not limited, that a 3D printer is used as the orthosis manufacturing apparatus 300.

As described above, according to the embodiment of the present invention, the reference model is generated by setting the reference points that become a reference of a joint structure or skeleton structure of a body part on which the orthosis is worn, the orthosis design is generated by mapping the orthosis design template to the reference model based on the reference points, and the orthosis can be manufactured just with the generated orthosis design.

Accordingly, the orthosis optimized to the orthosis wearer can be manufactured, and thus a reliability of manufacturing the orthosis can be maximized.

Further, since the orthosis design can be generated in a very short time through the orthosis design generating application and then the orthosis can be manufactured immediately using the orthosis manufacturing apparatus, manufacturing processes of the orthosis can be reduced and simplified considerably and thus an orthosis manufacturing efficiency can be maximized.

Meanwhile, it is obvious that the system and method of manufacturing the orthosis according to the above-described embodiment of the present invention are applied, in a same or similar way, to manufacturing orthoses for various parts such as human's four limbs (legs and arms), neck and the like. Further, it is obvious that the system and method are applied to manufacturing orthoses for not only a human but also an animal.

## Claims

1. An orthosis design manufacturing system, comprising:
an orthosis design generating system configured: to generate an information of a reference point using mark points displayed on a 3D image of an outer form for a body part of an examinee on which an orthosis is worn; to generate a reference model including an outer form information of the 3D image and the information of the reference point; and based on the information of the reference point, to map a selected orthosis design template to the reference model and generate an orthosis design,
wherein the information of the reference point includes an information of a joint reference point for a joint axis of the body part.

2. The orthosis design manufacturing system of claim 1, wherein the mark points includes a first mark point that is marked directly on the body part and is displayed on the 3D image, and a second mark point that is marked on the 3D image using the orthosis design generating system and is displayed on the 3D image.

3. The orthosis design manufacturing system of claim 1, wherein the orthosis design template includes an information of a standard reference point corresponding to the information of the reference point, and
wherein the standard reference point is matched to the reference point of the reference model, and based on this matching, the orthosis design template is mapped to the reference model.

4. The orthosis design manufacturing system of claim 3, wherein when matching a standard joint reference point to a joint reference point of the reference model, a joint axis of the standard joint reference point is matched to a joint axis of the joint reference point.

5. The orthosis design manufacturing system of claim 1, wherein the reference point includes an origin point of a coordinate system for the body part.

6. The orthosis design manufacturing system of claim 1, wherein the orthosis design generating system is configured to map the orthosis design template to the reference model and generate the orthosis design, based on an information of a bone line connecting the reference points neighboring to each other and the information of the reference point.

7. The orthosis design manufacturing system of claim 1, wherein the orthosis design generating system is configured to correct a posture of the reference model.

8. The orthosis design manufacturing system of claim 1, wherein the orthosis design generating system is configured to adjust a thickness for at least a region of the orthosis design.

9. The orthosis design manufacturing system of claim 1, wherein the body part is one of four limbs or a neck.

10. The orthosis design manufacturing system of claim 1, further comprising:
a 3D scanner that scans the body part and generates the 3D image; and
a 3D printer that receives an information of the orthosis design and manufactures the orthosis.

11. An orthosis design manufacturing method, comprising:
in an orthosis design generating system, generating an information of a reference point using mark points displayed on a 3D image of an outer form for a body part of an examinee on which an orthosis is worn; generating a reference model including an outer form information of the 3D image and the information of the reference point; and based on the information of the reference point, mapping a selected orthosis design template to the reference model and generating an orthosis design,
wherein the information of the reference point includes an information of a joint reference point for a joint axis of the body part.

12. The orthosis design manufacturing method of claim 11, wherein the mark points includes a first mark point that is marked directly on the body part and is displayed on the 3D image, and a second mark point that is marked on the 3D image using the orthosis design generating system and is displayed on the 3D image.

13. The orthosis design manufacturing method of claim 11, wherein the orthosis design template includes an information of a standard reference point corresponding to the information of the reference point, and
wherein the standard reference point is matched to the reference point of the reference model, and based on this matching, the orthosis design template is mapped to the reference model.

14. The orthosis design manufacturing method of claim 13, wherein when matching a standard joint reference point to a joint reference point of the reference model, a joint axis of the standard joint reference point is matched to a joint axis of the joint reference point.,

15. The orthosis design manufacturing method of claim 11, wherein the reference point includes an origin point of a coordinate system for the body part.

16. The orthosis design manufacturing method of claim 11, wherein in the orthosis design generating system, the orthosis design template is mapped to the reference model and the orthosis design is generated, based on an information of a bone line connecting the reference points neighboring to each other and the information of the reference point.

17. The orthosis design manufacturing method of claim 11, wherein in the orthosis design generating system, a posture of the reference model is corrected.

18. The orthosis design manufacturing method of claim 11, wherein in the orthosis design generating system, a thickness for at least a region of the orthosis design is adjusted.

19. The orthosis design manufacturing method of claim 11, wherein the body part is one of four limbs or a neck.

20. The orthosis design manufacturing method of claim 11, further comprising:
scanning the body part and generating the 3D image using a 3D scanner; and
receiving an information of the orthosis design and manufacturing the orthosis in a 3D printer.

## Amended claims

### Amended claims under Art. 19.1 PCT

1. An orthosis design manufacturing system, comprising:
an orthosis design generating system configured: to generate an information of a reference point using mark points displayed on a 3D image of an outer form for a body part of an examinee on which an orthosis is worn; to generate a reference model including an outer form information of the 3D image and the information of the reference point; and based on the information of the reference point, to map a selected orthosis design template to the reference model and generate an orthosis design,
wherein the information of the reference point includes an information of a joint reference point for a joint axis of the body part.

2. The orthosis design manufacturing system of claim 1, wherein the mark points includes a first mark point that is marked directly on the body part and is displayed on the 3D image, and a second mark point that is marked on the 3D image using the orthosis design generating system and is displayed on the 3D image.

3. The orthosis design manufacturing system of claim 1, wherein the orthosis design template includes an information of a standard reference point corresponding to the information of the reference point, and
wherein the standard reference point is matched to the reference point of the reference model, and based on this matching, the orthosis design template is mapped to the reference model.

4. The orthosis design manufacturing system of claim 3, wherein when matching a standard joint reference point to a joint reference point of the reference model, a joint axis of the standard joint reference point is matched to a joint axis of the joint reference point.

5. The orthosis design manufacturing system of claim 1, wherein the reference point includes an origin point of a coordinate system for the body part.

6. The orthosis design manufacturing system of claim 1, wherein the orthosis design generating system is configured to map the orthosis design template to the reference model and generate the orthosis design, based on an information of a bone line connecting the reference points neighboring to each other and the information of the reference point.

7. The orthosis design manufacturing system of claim 1, wherein the orthosis design generating system is configured to correct a posture of the reference model, and/or
wherein the orthosis design generating system is configured to adjust a thickness for at least a region of the orthosis design, and/or
wherein the body part is one of four limbs or a neck.

8. The orthosis design manufacturing system of claim 1, further comprising:
a 3D scanner that scans the body part and generates the 3D image; and
a 3D printer that receives an information of the orthosis design and manufactures the orthosis.

9. An orthosis design manufacturing method, comprising:
in an orthosis design generating system, generating an information of a reference point using mark points displayed on a 3D image of an outer form for a body part of an examinee on which an orthosis is worn; generating a reference model including an outer form information of the 3D image and the information of the reference point; and based on the information of the reference point, mapping a selected orthosis design template to the reference model and generating an orthosis design,
wherein the information of the reference point includes an information of a joint reference point for a joint axis of the body part.

10. The orthosis design manufacturing method of claim 9, wherein the mark points includes a first mark point that is marked directly on the body part and is displayed on the 3D image, and a second mark point that is marked on the 3D image using the orthosis design generating system and is displayed on the 3D image.

11. The orthosis design manufacturing method of claim 9, wherein the orthosis design template includes an information of a standard reference point corresponding to the information of the reference point, and
wherein the standard reference point is matched to the reference point of the reference model, and based on this matching, the orthosis design template is mapped to the reference model, and optionally,
wherein when matching a standard joint reference point to a joint reference point of the reference model, a joint axis of the standard joint reference point is matched to a joint axis of the joint reference point..

12. The orthosis design manufacturing method of claim 9, wherein the reference point includes an origin point of a coordinate system for the body part.

13. The orthosis design manufacturing method of claim 9, wherein in the orthosis design generating system, the orthosis design template is mapped to the reference model and the orthosis design is generated, based on an information of a bone line connecting the reference points neighboring to each other and the information of the reference point.

14. The orthosis design manufacturing method of claim 9, wherein in the orthosis design generating system, a posture of the reference model is corrected, and/or
wherein in the orthosis design generating system, a thickness for at least a region of the orthosis design is adjusted, and/or
wherein the body part is one of four limbs or a neck.

15. The orthosis design manufacturing method of claim 11, further comprising:
scanning the body part and generating the 3D image using a 3D scanner; and
receiving an information of the orthosis design and manufacturing the orthosis in a 3D printer.
